# FASCICULE DE BREVET EUROPEEN

(11) **EP 1 283 033 B1**
(45) Date de publication et mention de la délivrance du brevet: **20.02.2008**
(21) Numéro de dépôt: 02291875.9
(22) Date de dépôt: 24.07.2002
(51) Int. Cl.: A61K 8/49, A61K 8/67, A61Q 17/00, A61Q 19/00

(54) **Association d'alkylglycoside et de pyridinecarboxamide**
Mischung bestehend aus Alkylglycosid und Pyridincarboxamid
Combination of alkylglycoside and pyridine carboxamide

(30) Priorité: 24.07.2001 FR 0109859
(43) Date de publication de la demande: 12.02.2003
(73) Titulaire: CS, F-75008 Paris (FR)
(72) Inventeur: Rase, Didier, 75116 Paris (FR); Desmolin, Henri, 37550 Saint-Avertin (FR)
(74) Mandataire: Jappy, John William Graham

(56) Documents cités:
- EP-A- 0 185 971
- EP-A- 0 252 276
- WO-A-00/61066
- WO-A-95/09605
- WO-A-97/22346
- CA-A- 2 245 254
- FR-A- 2 740 039
- US-A- 5 416 075
- US-A- 6 080 323
- DATABASE CAPLUS [en ligne] CHEMICAL ABSTRACTS SERVICE, COLUMBUS, OHIO, US; retrieved from STN Database accession no. 1991:64784 XP002200571

## Description

### Domaine de l'invention

La présente invention a trait à une association d'alkyloside et de pyridinecarboxamide pour inhiber substantiellement la fixation de microorganismes de la famille des ***Propionibacterium acnes*** sur les cornéocytes. Elle concerne plus précisément l'utilisation topique de cette association ainsi qu'une composition dermatologique utile en cosmétique et/ou thérapeutique contenant au moins cette association.

### Art antérieur

Par produit inhibant la fixation d'un microorganisme sur une cellule humaine, animale ou végétale, on entend un produit qui (a) dénature ou inactive la protéine d'adhésion (également appelée "molécule d'adhésion") ou (b) se fixe sur le ou les récepteurs de ladite protéine d'adhésion présents sur la paroi de ladite cellule en empêchant, selon un mécanisme dit de compétition, la fixation de ladite protéine d'adhésion. Le processus microbiologique n'est pas encore clairement élucidé. On présumait autrefois, mais il s'agit là d'une théorie qui ne lie pas la Demanderesse, que le mécanisme pouvait comprendre :
(A) l'inactivation de la protéine d'adhésion, qui est en général un produit protéinique, ou la dénaturation de ladite protéine d'adhésion, ce qui conduit à l'absence ou à la limitation substantielle de la fixation de ladite protéine d'adhésion sur son ou ses récepteurs spécifiques ;
(B) la fixation de la substance inhibitrice sur le ou les récepteurs de la protéine d'adhésion ce qui empêche ou limite substantiellement, par compétition, la fixation de ladite protéine d'adhésion sur son ou ses récepteurs cellulaires ; ou,
(C) le cas échéant, une contribution de (A) plus (B).

On sait que les mono- et polyaldéhydes ont des propriétés antibactériennes et que, aux doses antibactériennes, ces produits ne sont pas tolérés par l'organisme : par application topique ils provoquent des irritations. Ceci explique pourquoi les mono- et polyaldéhydes sont utilisés à des doses plus faibles en tant que conservateurs dans les préparations cosmétiques et pharmaceutiques, voir à ce sujet WO-A-94/04167 et l'article de N. MELTZER et al., Cosmetics and Toileteries. 1977:124, 95-98*.*

Le brevet français délivré FR-B-2740039 préconise une solution technique très efficace pour inhiber la fixation des staphylocoques, en particulier les souches de ***Staphylococcus aureus*,** sur les cornéocytes. Cette solution met de préférence en oeuvre un dialdéhyde particulier, à savoir le glutaraldéhyde (ou 1,5-pentanedial) et convient vis-à-vis de la dermite atopique et des complications de cette maladie.

Dans l'article de S. J. MILLER et al., Arch. Dermatol., 1988:124, 209-215*,* il est recommandé de faire appel à un acide gras libre, de préférence extrait ou isolé du stratum corneum [où ledit acide est sous forme liée (en particulier sous une forme ester du type triglycéride)] pour protéger les cornéocytes de l'action des souches de ***Staphylococcus aureus***. Il se trouve que l'utilisation d'un acide gras est nettement insuffisante pour inhiber substantiellement *in vivo* la fixation desdites souches de ***Staphylococcus aureus**.*

Il existe cependant un problème qui vient de se manifester en liaison avec le point (C) ci-dessus. Les phénomènes d'adhésion et d'inhibition sont, sur le plan pharmacologique, particulièrement complexes. Pour inhiber la fixation des microorganismes, le produit inhibiteur doit se lier soit aux sites d'adhésion des cornéocytes, soit à ceux desdits microorganismes. Si ce produit se lie à la fois sur les deux types de sites, il va pouvoir se comporter comme une "colle" et augmenter l'adhésion des microorganismes aux cornéocytes. Ainsi selon les conditions expérimentales mises en oeuvre (notamment : durée d'incubation, concentration de la substance inhibitrice), l'effet inhibiteur d'un produit sur l'adhésion d'un microorganisme aux cornéocytes est donc susceptible de se transformer en un effet potentialisant l'adhésion que l'on veut éviter !

On sait, notamment des documents publiés WO-A-97/22346, US-A-6 080 323, EP-A-0 252 276, WO-A-95/09605, CA-A-2 245 254 et EP-A-0 185 971, que des alkylosides identiques ou similaires aux alkylosides de formule I ci-après selon l'invention sont des substances connues en tant qu'agents (i) tensioactifs et (ii) antimicrobiens (en particulier bactéricides, fongicides, virucides, désinfectants et/ou antiseptiques).

Le document WO-A-97/22346 enseigne le traitement topique de l'acné par destruction des souches responsables, à savoir les souches de ***Propionibacterium acnes*** (voir page 9 ligne 13 et page 13 ligne 17), au moyen d'un alkyloside, le PLANTAREN^{®} 1200 (voir Ex 2-4, 6 et 9-10) qui est un alkyloside de formule I ci-après, en association, le cas échéant, avec un autre principe (voir page 20 lignes 13-17) tel qu'un amide gras. Toutefois ce document ne décrit ni ne suggère l'association alkyloside/pyridinecarboxamide selon l'invention, d'une part, et son effet inhibiteur vis-à-vis de la fixation des souches de ***Propionibacterium acnes*** sur les cornéocytes.

Le brevet US-A-6 080 323 décrit l'utilisation d'alkylosides, en tant qu'agents tensioactifs et antibactériens, dans la décontamination des surfaces immergées dans des systèmes d'eau polluée, en vue d'éliminer les biofilms.

Dans le domaine de la désinfection, EP-A-0 252 276 propose des alkylosides en tant que potentialisateurs d'alcools et/ou d'acides, WO-A-95/09605 préconise une association alkyloside/halogénophénol, et EP-A-0 185 971 a trait à des alkylosides en tant que potentialisateurs de substances antiseptiques appartenant à l'ensemble des biguanides.

La demande canadienne publiée CA-A-2 254 245 décrit une association alkyloside/iode pour le traitement de la peau et des cheveux vis-à-vis de certaines dermatoses telles que dermatite séborrhéique, eczéma et psoriasis.

A côté des documents précités qui ont trait à des techniques détruisant les microbes, on connaît du brevet US-A-5 416 075 une émulsion (notamment du type huile-dans-eau) comprenant une phase aqueuse, une phase huileuse et un système émulsifiant contenant un composé amphiphile qui a un fragment biospécifique à l'extrémité de son groupe hydrophile, pour éviter la fixation de microorganismes sur une paroi. Ledit composé amphiphile est avantageusement un dérivé de sucre du type aldobionamide ou du type acide lipoteichoïque comportant plusieurs groupes phosphate et alanine (voir colonne 4 lignes 52-63, colonne 5 lignes 14-31, et colonne 7 lignes 2-19). Avec un tel composé amphiphile polyfonctionnel les risques précités de l'adhésion des microorganismes sur leurs récepteurs ne sont pas nuls. Il se trouve par ailleurs que ce brevet américain, comme l'art antérieur discuté plus haut, ne décrit ni ne suggère (i) l'association alkyloside/pyridinecarboxamide selon l'invention, (ii) son inhibition de la fixation de souches de ***Propionibacterium acnes*** sur les cornéocytes et (iii) son intérêt topique vis-à-vis de l'acné.

Enfin de la page 35 de WO 00/610066 A on connaît des compositions de shampoing comprenant un composant I, qui est un cocoglycoside (i. e. PLANTACARE^{®} 818) en tant qu'agent tensioactif non ionique [qui ne convient pas selon la présente invention], et un composant II, qui est le nicotinamide (i. e. 3-pyridinecarboxamide), en association avec un grand nombre d'autres ingrédients. Ce document ne vise pas, d'une part, l'utilisation de l'association alkylglycoside/pyridinecarboxamide pour inhiber la fixation des souches de ***Propionibacterium acnes*** sur les cornéocytes, et d'autre part, le rapport pondéral I/II, qui est de 1/1 à 1/4, de l'association et de la composition selon l'invention.

### But de l'invention

Il existe un besoin à satisfaire en ce qui concerne la protection des cornéocytes vis-à-vis des souches bactériennes, que sont les souches de ***Propionibacterium acnes*,** d'une part, et une solution technique pour pallier au problème susvisé de la potentialisation de l'adhésion, d'autre part.

On se propose de fournir une nouvelle solution faisant appel à une association particulière inhibant la fixation de ***Propionibacterium acnes*** sur les cornéocytes et qui conserve ses propriétés inhibitrices lorsque les conditions expérimentales varient.

### Objet de l'invention

Le but précité a été atteint grâce à une nouvelle solution technique mettant en oeuvre une association alkyloside/pyridinecarboxamide.

Selon un premier aspect de l'invention, on préconise une association pour usage topique en cosmétique ou thérapeutique (1°) d'une substance alkyloside constituée d'alkylglycosides, les alkylglycosides comprenant en poids, au moins 75% :
(i) d'alkylglycosides de formule I: 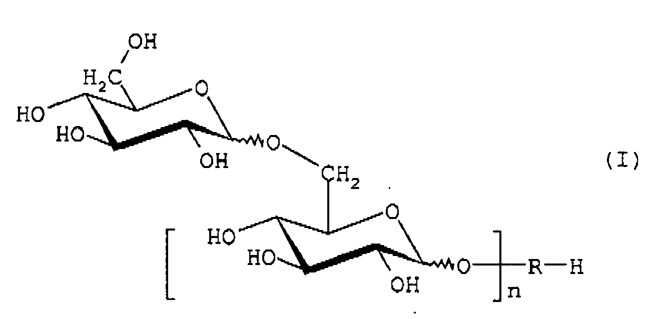 dans laquelle,
   n est un nombre ayant pour valeur 0 à 4, et
   R est (CH₂)₁₂ ou (CH₂)₁₄ ; et,
(ii) leurs mélanges,
avec (2°) au moins une substance appartenant à l'ensemble des pyridinecarboxamides de formule II : 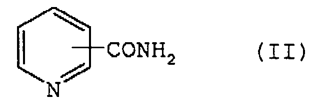 le rapport pondéral I/II étant compris entre 1/1 et 1/4, pour inhiber la fixation de souches de ***Propionibacterium acnes*** sur les cornéocytes.

Selon un second aspect de l'invention, on préconise une nouvelle composition pour usage topique en cosmétique ou thérapeutique, ladite composition étant caractérisée en ce qu'elle comprend, en association avec un excipient dermatologiquement acceptable, (1°) une substance alkyloside constituée d'alkylglycosides, les alkylglycosides comprenant en poids, au moins 75% :
(i) d'alkylglycosides de formule I: 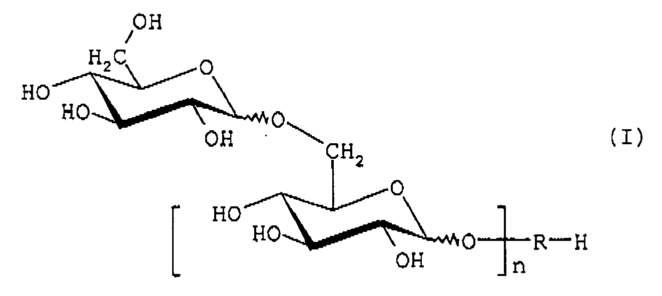 dans laquelle,
   n est un nombre ayant pour valeur 0 à 4, et
   R est (CH₂)₁₂ ou (CH₂)₁₄ ; et,
(ii) leurs mélanges,
et (2°) au moins une substance appartenant à l'ensemble des pyridinecarboxamides de formule II : 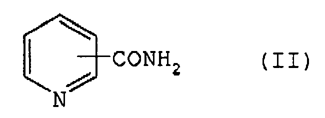 le rapport pondéral I/II étant compris entre 1/1 et 1/4, pour inhiber la fixation de souches de ***Propionibacterium acnes*** sur les cornéocytes.

Selon un troisième aspect de l'invention, on préconise une nouvelle utilisation de l'association d'une substance alkyloside constituée d'alkylglycosides, les alkylglycosides comprenant en poids, au moins 75% :
(i) d'alkylglycosides de formule I: 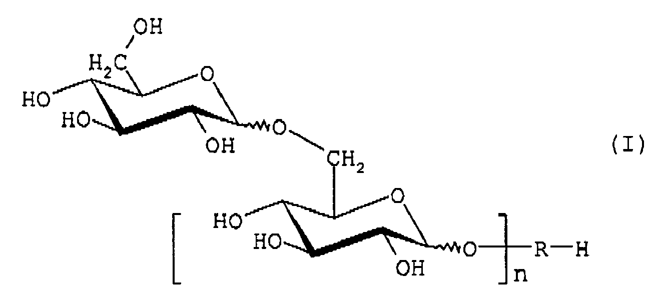 dans laquelle,
   n est un nombre ayant pour valeur 0 à 4, et
   R est (CH₂)₁₂ ou (CH₂)₁₄ ; et,
(ii) leurs mélanges,
avec au moins une substance appartenant à l'ensemble des pyridinecarboxamides de formule II : 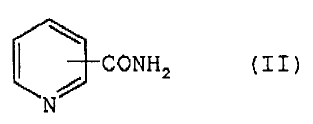 le rapport pondéral I/II étant compris entre 1/1 et 1/4, pour la préparation d'une composition destinée à un usage local en cosmétique ou en thérapeutique vis-à-vis de l'acné induit par la fixation desdites souches de ***Propionibacterium acnes*** sur les cornéocytes.

Dans cette utilisation, le rapport pondéral I/II est avantageusement compris entre 1/1 et 1/4, et mieux entre 2/3 et 3/4.

### Description détaillée de l'invention

Des mélanges d'alkylglycosides contenant des produits de formule I sont déjà commercialisés notamment sous les noms de marque de PLANTACARE^{®} et de PLANTAREN^{®} et chaque alkylglycoside de ces mélanges peut être synthétisé selon une méthode opératoire connue en soi par application de mécanismes réactionnels classiques.

Selon l'invention, il est important que R soit une chaîne hydrocarbonée linéaire en C₁₂ ou C₁₄ ou que, dans le cadre d'un mélange d'alkylglycosides, la teneur en composés C₁₂-C₁₄ soit supérieure ou égale à 75 % en poids (de préférence supérieure ou égale à 80 % en poids) par rapport au poids dudit mélange.

Si R représente (CH₂)₈ ou (CH₂)₁₀, chaque composé alkylglycoside résultant est inactif ou insuffisamment actif pour inhiber l'adhésion des ***Propionibacterium acnes*** sur les sites d'adhésion des cornéocytes. Il en est de même quand R est une chaîne hydrocarbonée linéaire en C₁₆ [i.e. (CH₂)₁₆] ou supérieure à C₁₆.

Le "PLANTACARE^{®} 1200", qui est un mélange comprenant :

| | |
|---|---|
| produits à chaîne linéaire ≤ C₁₀ | 0 à 2% en poids, |
| produits à chaîne linéaire ≥ C₁₆ | 0 à 6 % en poids et |
| produit à chaîne linéaire en C₁₂-C₁₄ | 100 à 92 % en poids, |

convient en tant que substance inhibant, en association avec une substance pyridinecarboxamide, la fixation de ***Propionibacterium acnes*** sur les cornéocytes humains.

En revanche, les mélanges commercialisés sous les nomenclatures de "PLANTACARE^{®} 2000" et "PLANTACARE^{®} 818" ne conviennent pas selon l'invention (aux même doses que le PLANTACARE^{®} 1200) eu égard à leurs compositions :

| Produits à chaîne linéaire | "PLANTACARE^{®} 2000" | "PLANTACARE^{®} 818" |
|---|---|---|
| ≤ C₁₀ | 60 à 75 % en poids | 39 à 53 % en poids |
| ≥ C₁₆ | 0 à 1 % en poids | 0 à 4 % en poids |
| C₁₂-C₁₄ | 40 à 24 % en poids | 61 à 43 % en poids |

De façon avantageuse, R-H sera un groupe lauryle [(CH₂)₁₂H] plutôt qu'un groupe myristyle [(CH₂)₁₄H].

De façon également avantageuse, n aura pour valeur 0, 1 ou 2. Il convient toutefois de remarquer, que lorsque l'on utilise un mélange (tel que le PLANTACARE^{®} précité), on est en présence d'une association de plusieurs molécules de formule I et n devient globalement un nombre fractionnaire avantageusement compris entre 0,2 et 1,6.

Les alkylglycosides de formule I selon l'invention comprennent notamment
- les α- et β-alkylgalactosides de formule : 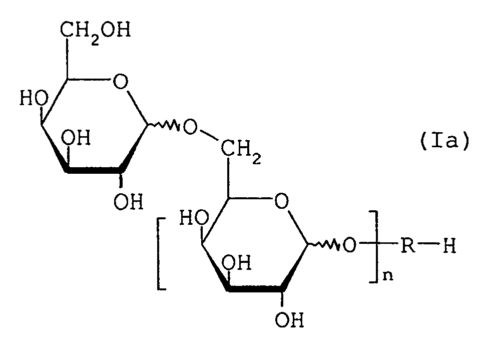
- les α- et β-alkylglucosides de formule : 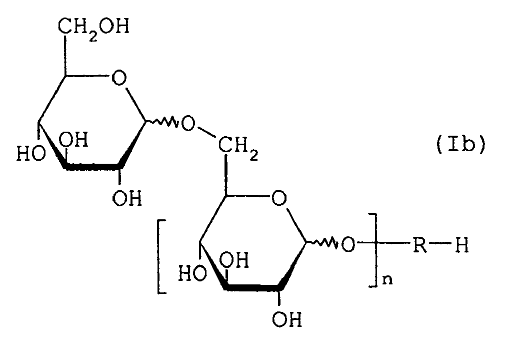 et
- les α- et β-alkylmannosides de formule :

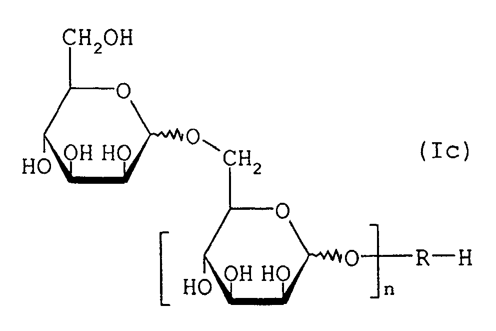

De façon pratique, on préfère plutôt les composés alkylglycosides de formule Ib [i.e. les α- et β-alkylglucosides] à ceux des formules Ia et Ic.

Les pyridinecarboxamides de formule II ci-dessus comprennent les 2-pyridinecarboxamide (picolinamide), 3-pyridinecarboxamide (nicotinamide) et 4-pyridinecarboxamide (isonicotinamide) de formules respectives IIa, IIb et IIc:

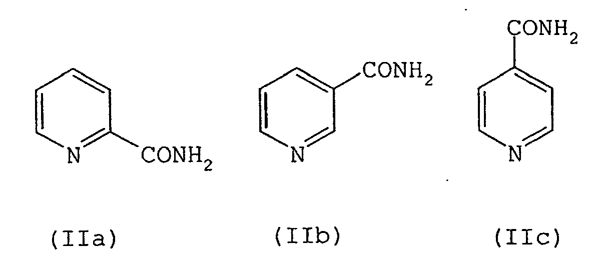

Le pyridinecarboxamide préféré selon l'invention est le 3-pyridinecarboxamide.

Les substances de formule I et leurs associations avec les pyridinecarboxamides de formule II sont efficaces pour inhiber la fixation des souches de ***Propionibacterium acnes.*** En particulier on a constaté que les alkylglycosides de formule I en association avec les pyridinecarboxamides de formule II bloquent les sites récepteurs des ***Propionibacterium acnes*.** Après administration de ces produits par voie topique (notamment sous forme de gel), la quantité des souches précitées qui va se fixer est nettement moindre en cas d'acné dû auxdites souches de ***Propionibacterium acnes*.**

La composition selon l'invention présente une synergie en ce qui concerne l'inhibition de la fixation de ***Propionibacterium acnes*** sur les cornéocytes. Cette synergie est particulièrement notable quand le rapport pondéral I/II est compris entre 1/1 et 1/4.

Ladite composition comprendra avantageusement (1°) 1 à 2 parties en poids d'une substance choisie parmi les alkylglycosides de formule I et leurs mélanges, pour (2°) 1 à 4 parties en poids d'une substance choisie parmi les pyridinecarboxamides de formule II et leurs mélanges.

De façon pratique le rapport pondéral particulièrement préféré I/II sera compris entre 2/3 et 3/4 (la synergie est alors maximale), la composition correspondante de l'invention pouvant alors contenir notamment 4 % en poids de (1°) et 6 % en poids de (2°), ou 3 % en poids de (1°) et 4 % en poids de (2°).

D'autres avantages et caractéristiques de l'invention seront mieux compris à la lecture qui va suivre d'exemples de réalisation et de résultats d'essais. Bien entendu l'ensemble de ces éléments n'est pas limitatif mais est donné à titre d'illustration.

### Exemple 1

On prépare une composition sous forme de gel ayant la formulation (en % en poids) suivante :

| | |
|---|---|
| laurylglucoside (PLANTACARE^{®} 1200) | 3,00 % |
| nicotinamide | 4,00 % |
| éthanol | 22,75 % |
| polyoxyéthylène alcool (laureth-12) | 6,00 % |
| aluminosilicate de magnesium (VEEGUM^{®} K) | 2,00 % |
| hydroxypropyl-méthylcellulose | 1,70 % |
| acide citrique | 0,15 % |
| eau | qsp 100,00 % |

### Exemple 2

Avec les excipients de l'exemple 1, on prépare une composition sous forme de gel contenant :

| | |
|---|---|
| laurylglucoside (formule I, n = 0,6) | 4,00 % |
| nicotinamide | 6,00 % |

Remarque : ici le laurylglucoside de formule I où n est 0,6 est obtenu par mélange de 1,3 moles de laurylglucoside de formule I où n est 0 avec 2 moles de laurylglucoside de formule I où n est 1.

### Exemple 3

Avec les excipients de l'exemple 1, on prépare une composition sous forme de gel contenant :

| | |
|---|---|
| laurylglucoside (formule I, n = 1,6) | 3,5% |
| nicotinamide | 5,0 % |

Remarque : ici le laurylglucoside de formule I où n est 1,6 est obtenu par mélange de 1,3 moles de laurylglucoside de formule I où n est 1 avec 2 moles de laurylglucoside de formule I où n est 2.

### Exemple 4

Avec les excipients de l'exemple 1, on prépare une composition sous forme de gel contenant :

| | |
|---|---|
| laurylglucoside (PLANTACARE^{®} 1200) | 4,0 % |
| isonicotinamide | 6,0 % |

On a résumé ci-après une partie des essais qui ont été entrepris.

### Essais ex vivo

### Produits

Les produits suivants ont été utilisés.
Produit à tester : composition de l'exemple 1,
tampon : PB S [produit contenant NaCl(8 g/l), Na₂HPO₄ (1,15 g/l), KH₂PO₄ (0,2 g/l), KCl (0,2 g/l), CaCl₂ (0,1 g/l) et MgCl₂ (0,1 g/l) ; pH 7,4],
dispersant : Tween 80 (fabriqué par la société SIGMA),
eau : eau ultrapure (selon système de filtration MILLIQ^{®} de la société MILLIPORE),
souche bactérienne : ***Propionibacterium acnes,*** souche colorée à l'acrydine orange.

### Traitement

Les sujets acnéiques et non acnéiques ont été traités pendant 3 jours (application 1 fois par jour de l'exemple 1 à tester) sur un avant-bras, l'autre avant-bras servant de contrôle. L'usage d'un manchon plastifié est intervenu pour protéger chaque avant-bras pendant la toilette.

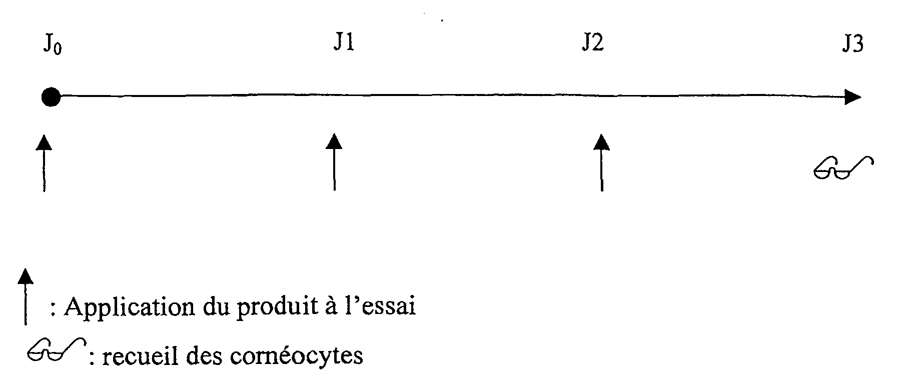

Pour la préparation des cornéocytes, on a d'abord procédé au prélèvement par lavage des avant-bras par une solution de Tween 1 % dans du PBS puis au recueil des eaux de lavage. Ensuite, les cellules (i.e. cornéocytes) contenues dans les eaux de lavage ont été recueillies après filtration sur toile nylon (maille 60 µm). Enfin elles ont été lavées par centrifugation (10 min à 1000 tours/min ; 3 lavages successifs) dans du PBS. Une nouvelle filtration est intervenue avant comptage des amas de cornéocytes dans une cellule de Nageotte.

### Incubation

Les suspensions de cornéocytes ont été incubées en présence de ***Propionibacterium acnes*** pendant 1 h sous agitation rotative douce, à l'abri de la lumière, et à température ambiante (RT = 15-25°C).

### Mesure

On a déterminé par cytométrie en flux la variation d'intensité moyenne de fluorescence présente à la surface des cellules ayant fixé les bactéries (FACScalibur^{®} ; Sté Becton Dickinson), avec excitation à 500 nm et émission à 530 nm, et analyse des données au moyen du logiciel CellQuest^{®}.

La fluorescence des cornéocytes seuls ("blanc") a été mesurée et soustraite des valeurs de fluorescence des échantillons mesurés.

Pour l'expression des résultats, on a retenu les unités de fluorescence par échantillon et le pourcentage de variation par rapport au groupe dit à "zone non traitée", eu égard à celle du groupe dit à "zone traitée" correspondant.

### Résultats

Les résultats obtenus sont consignés ci-après dans les tableaux I [sujets acnéiques (hommes) C et D], II [sujets acnéiques 6-8 (femmes) et 9-10 (hommes)] et III [tableau récapitulatif pour lesdits sujets acnéiques 6-10], la notation de la signification statistique étant fondée sur le "barême"
* = moyenne significativement différente de celle du groupe témoin correspondant, p < 0,1
** = moyenne significativement différente de celle du groupe témoin correspondant, p < 0,05
*** = moyenne significativement différente de celle du groupe témoin correspondant, p < 0,01
les résultats de l'étude *ex vivo* étant tous notés ici *** (i.e. p ≤ 0,01).

**TABLEAU I**

| ***Effet ex vivo de l'Exemple 1 sur l'adhésion de Propionibacterium acnes aux cornéocytes humains (sujets acnéiques) - 1er lot -*** | | | | |
|---|---|---|---|---|
| | Sujet C acnéique (homme) | | Sujet D acnéique (homme) | |
| | Cornéocytes non traités | Cornéocytes traités | Cornéocytes non traités | Cornéocytes traités |
| blanc | 18,1 | 9,1 | 4,2 | 7,1 |
| (fluorescence - blanc) | 3243,3 | 603,6 | 1528,2 | 61,9 |
| | 3329,1 | 590,0 | 1500,9 | 59,3 |
| | 3164,4 | 571,7 | 1453,1 | 59,1 |
| **moyenne** | **3245,6** | **588,4***** | **1494,1** | **60,1***** |
| écart type | 82,3 | 16,0 | 38,0 | 1,5 |
| **% témoin** | **100** | **18** | **100** | **4** |

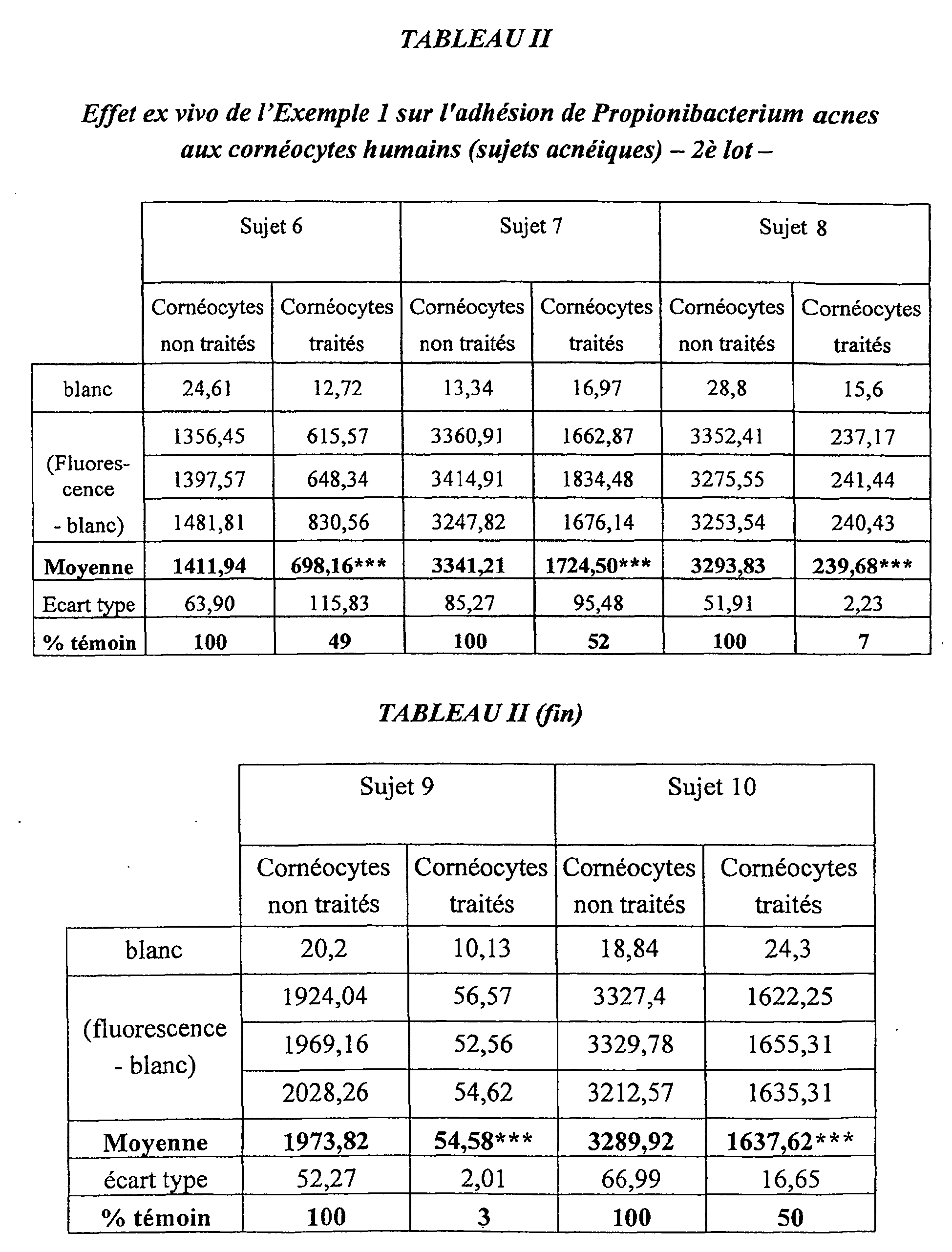

Dans cette étude, les quantités du gel de Ex 1, administrées (par voie topique pendant les 3 jours de traitement) aux sujets 6-10, étaient de 2,14 g pour le sujet 6, de 1,41 g pour le sujet 7, de 2,89 g pour le sujet 8, de 2,75 g pour le sujet 9 et de 2,56 g pour le sujet 10.

**TABLEAU III**

| **Effet inhibiteur de l'Exemple 1 sur l'adhésion de *Propionibacterium acnes* aux cornéocytes humains (sujets acnéiques) - récapitulation** | |
|---|---|
| Ex 1 | |
| Sujets acnéiques | % d'inhibition |
| 6 (femme) | 51 % |
| 7 (femme) | 48 % |
| 8 (femme) | 93 % |
| 9 (homme) | 97 % |
| 10 (homme) | 50 % |
| **Moyenne** | **67,8** |
| Ecart-type | 24,9 |

### Essais in vitro

En suivant les modalités opératoires données ci-dessus, en ce qui concerne le prélèvement des cornéocytes, l'incubation avec la souche de ***Propionibacterium acnes*** et la mesure des fluorescences, on a comparé l'adhésion in vitro de ladite souche aux cornéocytes humains prélevés chez un sujet sain [sujet B non acnéique] et chez des sujets acnéiques [sujets A, C et D (les sujets C et D étant ceux du 1er lot ci-dessus)] non traités. Les résultats obtenus sont consignés dans le tableau IV qui suit.

**TABLEAU IV**

| **Comparaison de l'adhésion *in vitro* de *Propionibacterium acnes* aux cornéocytes humains prélevés chez un sujet sain et chez des sujets acnéiques (cornéocytes non traités)** | | | | |
|---|---|---|---|---|
| | Sujet B non acnéique | Sujet A acnéique | Sujet C acnéique | Sujet D acnéique |
| Blanc | 11,1 | 13,1 | 18,1 | 4,2 |
| Fluorescence -blanc | 3629,8 | 4952,3 | 3243,3 | 1528,2 |
| | 3659,0 | 4737,5 | 3329,1 | 1500,9 |
| | 3679,9 | 4811,4 | 3164,4 | 1453,1 |
| **Moyenne** | **3656,2** | **4833,8** | **3245,6** | **1494,1** |
| Ecart type | 25,2 | 109,1 | 82,3 | 38,0 |

### Conclusions

Il résulte de l'ensemble de ces essais que :
1) l'adhésion *ex vivo* de ***Propionibacterium acnes*** aux kératinocytes de sujets acnéiques traités *in vivo* par le gel de l'exemple 1 est fortement diminuée ; et,
2) il semble [mais ceci doit être confirmé avec un nombre plus important de sujets (non acnéiques et acnéiques)] qu'il n'y aurait pas de différence d'adhésion de ***Propionibacterium acnes*** aux cornéocytes prélevés chez le sujet sain B ou chez les sujets acnéiques A, C et D.

## Revendications

1. Utilisation d'une substance alkyloside constituée d'alkylglycosides, les alkylglycosides comprenant en poids, au moins 75 % :
(i) d'alkylglycosides de formule I : 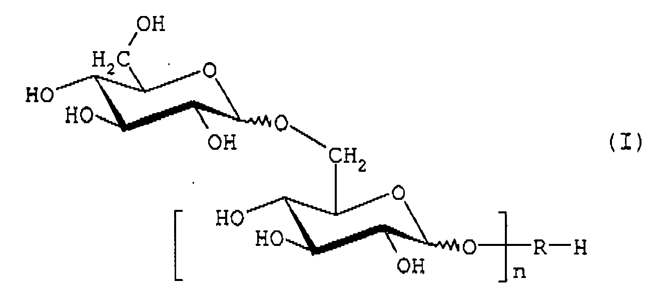 dans laquelle,
n est un nombre ayant pour valeur 0 à 4, et
R est (CH₂)₁₂ ou (CH₂)₁₄ ; ou,
(ii) du mélange de tels alkylglycosides de formule I,
et, d'au moins une substance appartenant à l'ensemble des pyridinecarboxamides de formule II : 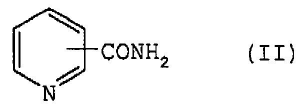 le rapport pondéral I/II étant compris entre 1/1 et 1/4,
pour la préparation d'une composition destinée à un usage local en cosmétique ou en thérapeutique vis-à-vis de l'acné induit par la fixation de souches de ***Propionibacterium acnes*** sur les cornéocytes.

2. Utilisation suivant la revendication 1, dans laquelle le rapport pondéral I/II est compris entre 2/3 et 3/4.

3. Utilisation suivant la revendication 1, **caractérisée en ce que** n est un nombre fractionnaire compris entre 0,2 et 1,6.

4. Association pour usage topique en cosmétique ou thérapeutique (1°) d'une substance alkyloside constituée d'alkylglycosides, les alkylglycosides comprenant en poids, au moins 75%:
(i) d'alkylglycosides de formule I : 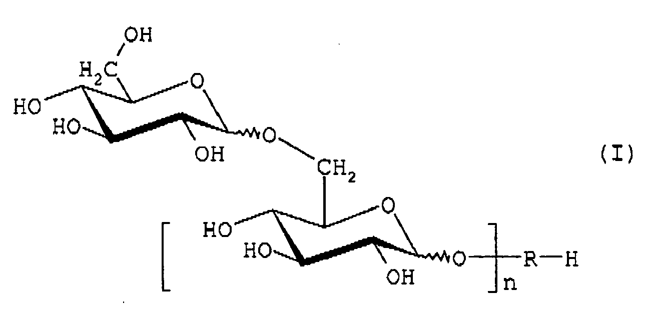 dans laquelle,
n est un nombre ayant pour valeur 0 à 4, et
R est (CH₂)₁₂ ou (CH₂)₁₄; et,
(ii) leurs mélanges,
avec (2°) au moins une substance appartenant à l'ensemble des pyridinecarboxamides de formule II : 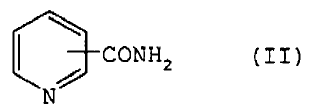 le rapport pondéral I/II étant compris entre 1/1 et 1/4,
pour inhiber la fixation de souches de ***Propionibacterium acnes*** sur les cornéocytes.

5. Association suivant la revendication 4, **caractérisée en ce que** n a pour valeur 0, 1 ou 2.

6. Association suivant la revendication 4, **caractérisée en ce que** n est un nombre fractionnaire compris entre 0,2 et 1,6.

7. Association suivant la revendication 4, dans laquelle le rapport pondéral I/II est compris entre 2/3 et 3/4.

8. Composition pour usage topique en cosmétique ou thérapeutique, ladite composition étant **caractérisée en ce qu'**elle comprend, en association avec un excipient dermatologiquement acceptable,
(1°) une substance alkyloside constituée d'alkylglycosides, les alkylglycosides, comprenant en poids, au moins 75%:
(i) d'alkylglycosides de formule I : 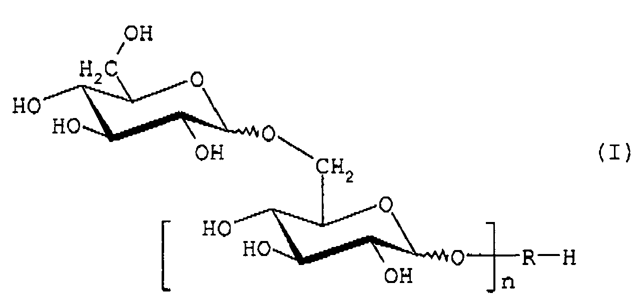 dans laquelle,
n est un nombre ayant pour valeur 0 à 4, et
R est (CH₂)₁₂ ou (CH₂)₁₄ ; et,
(ii) leurs mélanges,
et (2°) au moins une substance appartenant à l'ensemble des pyridinecarboxamides de formule II: 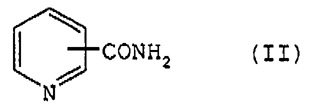 le rapport pondéral I/II étant compris entre 1/1 et 1/4,
pour inhiber la fixation de souches de ***Propionibacterium acnes*** sur les cornéocytes.

9. Composition suivant la revendication 8, **caractérisée en ce que** n a pour valeur 0, 1 ou 2.

10. Composition suivant la revendication 8, **caractérisée en ce que** n est un nombre fractionnaire compris entre 0,2 et 1,6.

11. Composition suivant la revendication 8, dans laquelle le rapport pondéral I/II est compris entre 2/3 et 3/4.

12. Composition suivant l'une quelconque des revendications 8 à 11, dans laquelle la substance alkyloside est un alkylgalactoside, alkylglucoside ou alkylmannoside, et en ce que la substance pyridinecarboxamide est le 3-pyridinecarboxamide.

## Claims

1. Use of an alkyloside substance formed of alkylglycosides, the alkylglycosides comprising by weight at least 75%:
(i) of alkylglycosides of formula I: 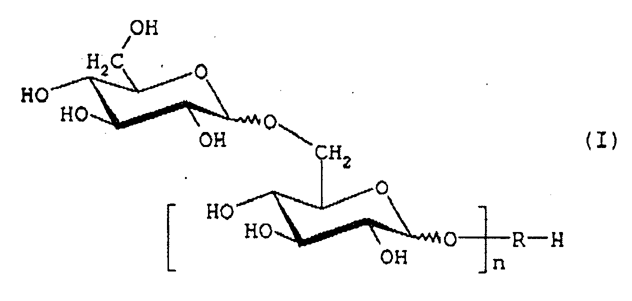 in which,
n is a number having a value of 0 to 4, and
R is (CH₂)₁₂ or (CH₂)₁₄; or
(ii) of the mixture of such alkylglycosides of formula I and at least one substance belonging to the group of pyridinecarboxamides of formula II: 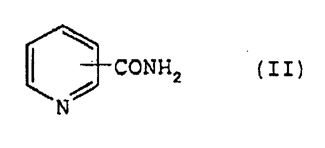
the weight ratio I/II being between I/I and 1/4, for the preparation of a composition intended for cosmetic or therapeutic topical use against acne induced by fixing of strains of ***Propionibacterium acnes*** to corneocytes.

2. Use as described in claim 1, in which the weight ratio I/II is between 2/3 and 3/4.

3. Use as described in claim 1, **characterised by** the fact that n is a fractional number between 0.2 and 1.6.

4. Association for cosmetic or therapeutic topical use (1^{st}) of an alkyloside substance formed of alkylglycosides, the alkylglycosides comprising by weight at least 75%:
(i) of alkylglycosides of formula I: 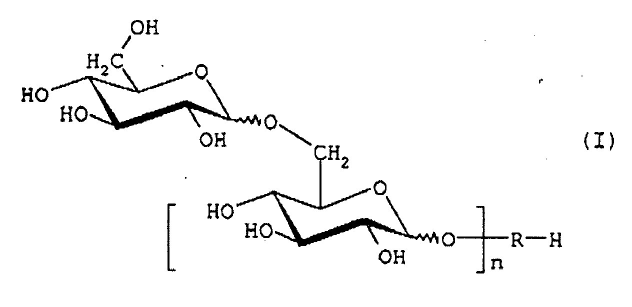 in which
n is a number having a value of 0 to 4; and
R is (CH₂)₁₂ or (CH₂)₁₄; and
(ii) their mixtures,
with at least one substance belonging to the group of pyridinecarboxamides of formula II: 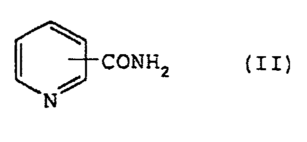 the weight ratio I/II being between 1/1 and 1/4,
to inhibit fixing of strains of ***Propionibacterium acnes*** to corneocytes.

5. Association as described in claim 4, **characterised by** the fact that n has the value of 0, 1 or 2.

6. Association as described in claim 4, **characterised by** the fact that n is a fractional number between 0.2 and 1.6.

7. Association as described in claim 4, in which the weight ratio I/II is between 2/3 and 3/4.

8. Composition for cosmetic or therapeutic topical use, the said composition being **characterised by** the fact that it comprises, in association with a dermatologically acceptable excipient,
(1^{st}) an alkyloside substance formed of alkylglycosides, the alkylglycosides comprising by weight at least 75%:
(i) of alkylglycosides of formula I: 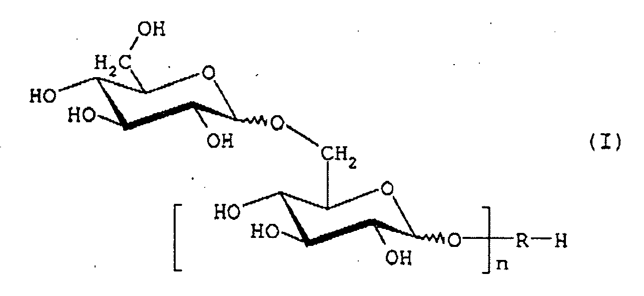 in which
n is a number having a value of 0 to 4; and
R is (CH₂)₁₂ or (CH₂)₁₄; and
(ii) their mixtures,
and (2^{nd}) at least one substance belonging to the group of pyridinecarboxamides of formula II: 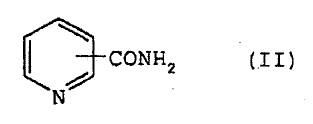 the weight ratio I/II being between 1/1 and 1/4,
to inhibit fixing of strains of ***Propionibacterium acnes*** to corneocytes.

9. Composition as described in claim 8, **characterised by** the fact that n has a value of 0, 1 or 2.

10. Composition as described in claim 8, **characterised by** the fact that n is a fractional number between 0.2 and 1.6.

11. Composition as described in claim 8, in which the weight ratio I/II is between 2/3 and 3/4.

12. Composition as described in any one of claims 8 to 11, in which the alkyloside substance is an alkylgalactoside, alkylglucoside, or alkylmannoside, and by the fact that the pyridinecarboxamide substance is 3-pyridinecaboxamide.

## Patentansprüche

1. Verwendung einer Alkylosidsubstanz, bestehend aus Alkylglycosiden, wobei die Alkylglycoside, bezogen auf das Gewicht, mindestens 75%:
(i) Alkylglycoside der Formel I: 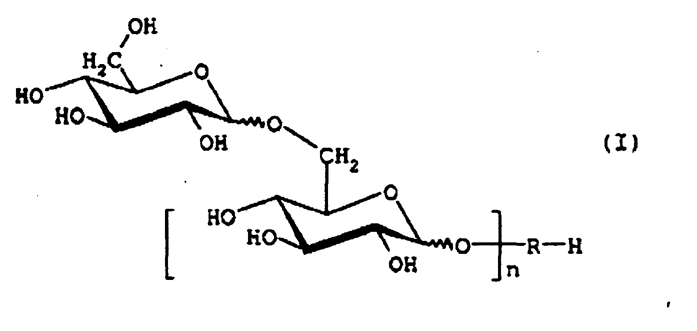 in welcher
n eine Zahl mit einem Wert von 0 bis 4 ist, und
R (CH₂)₁₂ oder (CH₂)₁₄ ist; oder
(ii) eines Gemischs der Alkylglycoside der Formel I umfassen,
und mindestens einer Substanz, die aus der Gruppe der Pyridincarboxamide der Formel II ausgewählt ist: 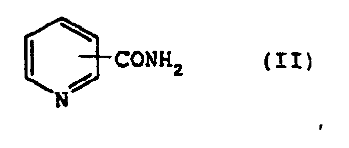 wobei das Gewichtsverhältnis von I/II zwischen 1/1 und 1/4 liegt,
zur Herstellung einer Zusammensetzung zur lokalen kosmetischen oder therapeutischen Verwendung gegen Akne, die durch eine Anlagerung von Stämmen von ***Propionibacterium acnes*** auf Corneocyten hervorgerufen wird.

2. Verwendung nach Anspruch 1, wobei das Gewichtsverhältnis von I/II zwischen 2/3 und 3/4 liegt.

3. Verwendung nach Anspruch 1, wobei n eine Bruchzahl zwischen 0,2 und 1,6 ist.

4. Zusammenschluss zur topischen kosmetischen oder therapeutischen Verwendung von (1) einer Alkylosidsubstanz, bestehend aus Alkylglycosiden, wobei die Alkylglycoside, bezogen auf das Gewicht, mindestens 75%:
(i) Alkylglycoside der Formel I: 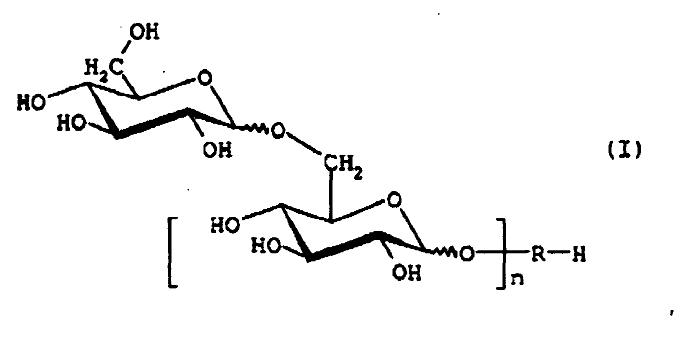 in welcher
n eine Zahl mit einem Wert von 0 bis 4 ist, und
R (CH₂)₁₂ oder (CH₂)₁₄ ist; und
(ii) ihrer Gemische umfassen,
mit (2) mindestens eine Substanz, die aus der Gruppe der Pyridincarboxamide der Formel II: 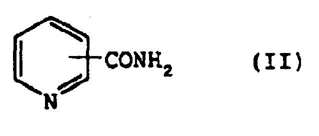 ausgewählt ist,
wobei das Gewichtsverhältnis von I/II zwischen 1/1 und 1/4 liegt,
zur Inhibition der Anlagerung von Stämmen von ***Propionibacterium acnes*** auf Corneocyten.

5. Zusammenschluss nach Anspruch 4, wobei n den Wert 0,1 oder 2 aufweist.

6. Zusammenschluss nach Anspruch 4, wobei n eine Bruchzahl zwischen 0,2 und 1,6 ist.

7. Zusammenschluss nach Anspruch 4, wobei das Gewichtsverhältnis von I/II zwischen 2/3 und 3/4 liegt.

8. Zusammensetzung zur topischen kosmetischen oder therapeutischen Verwendung, wobei die Zusammensetzung **dadurch gekennzeichnet ist, dass** sie in Verbindung mit einem dermatologisch erträglichen Hilfsstoff
(1) eine Alkylosidsubstanz, bestehend aus Alkylglycosiden, wobei die Alkyglycoside, bezogen auf das Gewicht, mindestens 75%
(i) Alkylglycoside der Formel I: 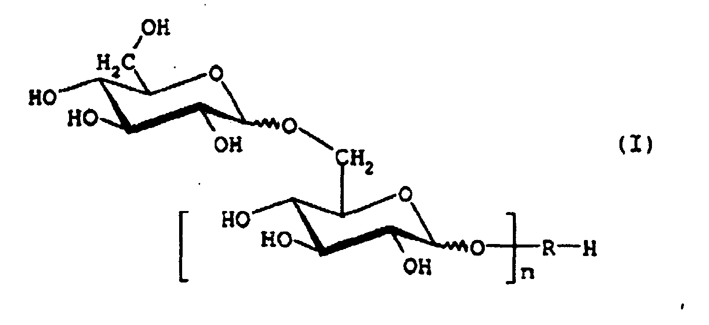 in welcher
n eine Zahl mit einem Wert von 0 bis 4 ist, und
R (CH₂)₁₂ oder (CH₂)₁₄ ist; und
(ii) ihrer Gemische umfassen, und
(2) mindestens eine Substanz, die aus der Gruppe der Pyridincarboxamide der Formel II: 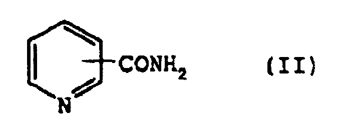
ausgewählt ist, umfasst,
wobei das Gewichtsverhältnis von I/II zwischen 1/1 und 1/4 liegt,
zur Inhibition der Anlagerung von Stämmen von ***Propionibacterium acnes*** auf Corneocyten.

9. Zusammensetzung nach Anspruch 8, wobei n einen Wert von 0,1 oder 2 aufweist.

10. Zusammensetzung nach Anspruch 8, wobei n eine Bruchzahl zwischen 0,2 und 1,6 ist.

11. Zusammensetzung nach Anspruch 8, wobei das Gewichtsverhältnis von I/II zwischen 2/3 und 3/4 liegt.

12. Zusammensetzung nach einem der Ansprüche 8 bis 11, wobei die Alkylosidsubstanz ein Alkylgalactosid, Alkylglucosid oder ein Alkylmannosid ist und die Pyridincarboxamidsubstanz 3-Pyridincarboxamid ist.
